# EUROPÄISCHE PATENTSCHRIFT

(11) **EP 2 389 975 B1**
(45) Veröffentlichungstag und Bekanntmachung des Hinweises auf die Patenterteilung: **22.08.2012**
(21) Anmeldenummer: 10005453.5
(22) Anmeldetag: 26.05.2010
(51) Int. Cl.: A61N 1/05, A61N 1/36

(54) **Implantierbare Sammelelektrode sowie Neurostimulationssystem**
Implantable electrode array and neurostimulation system
Electrode de collecte implantable et système de neurostimulation

(43) Veröffentlichungstag der Anmeldung: 30.11.2011
(73) Patentinhaber: Possover, Marc, Prof. Dr., 6032 Hagendorn (CH)
(72) Erfinder: Possover, Marc, Prof. Dr., 6032 Hagendorn (CH)
(74) Vertreter: Wagner, Kilian

(56) Entgegenhaltungen:
- WO-A1-2009/097224
- US-A1- 2007 255 383
- US-A1- 2010 100 152
- US-B1- 7 676 275

## Beschreibung

Die Erfindung betrifft eine in den menschlichen Körper laparoskopisch implantierbare, drahtförmige, Sammelelektrode zur Neurostimulation von Nerven oder als Sensor zur Detektion von Nervenimpulsen, umfassend mehrere einzeln und/oder in Gruppen kontaktierbare Mantelsegmentelektroden, die axial in Richtung der Längserstreckung der Sammelelektrode hintereinander angeordnet sind, wobei axial zwischen jeweils zwei benachbarten Sammelsegmentelektroden ein isolierender Abschnitt zum elektrischen voneinander Isolieren der jeweils zwei benachbarten Mantelsegmentelektroden angeordnet ist.

Es ist bekannt, zur Neurostimulation von Nerven (z.B. plexus sacralis, nervus ischiadicus, nervus pudendus, nervus femoralis) im kleinen Becken Sammelelektroden mit acht axial voneinander beabstandeten Mantelsegmentelektroden einzusetzen, die üblicherweise laparaskopisch implantiert werden. Dabei ist die Sammelelektrode über ein Anschlusskabel mit einem Schrittmacher verbunden, der die Sammelelektrode mit einem Stimulationsmuster beaufschlagt und dabei die Mantelsegmentelektroden einzeln ansteuert, um den gewünschten Nerv selektiv zu stimulieren. Bei den bekannten Sammelelektroden sind die jeweils benachbarten Mantelsegmentelektroden gleich beabstandet und zwar über jeweils einen, zwei benachbarte Mantelsegmentelektroden elektrisch voneinander isolierenden Abschnitt, wobei sämtliche isolierende Abschnitte die gleiche Axialerstreckung aufweisen. Das Problem bei der Implantation der Sammelelektrode ist die korrekte Positionierung der Sammelelektrode auf dem zu stimulierenden Nerv. Bei nicht optimaler Platzierung kommt es vor, dass der gewünschte Stimulationseffekt nicht eintritt und schlimmstenfalls das Patientenleiden sogar verschlimmert wird.

Aus der US 7,676,275 B1 ist eine Sammelelektrode zur Implantation im Herzen bekannt. In der Druckschrift ist in Fig. 5 eine Sammelelektrode gezeigt, bei der der Abstand zwischen einer vierten und einer fünften Mantelsegmentelektrode, gezählt vom distalen Ende her, größer ist als der Abstand zwischen zwei weiteren Mantelsegmentelektroden. Der geschaffene Freiraum zwischen der vierten und fünften Elektrode dient zur Festlegung eines Stützringes zum Stützen einer Aderwand im Herzen.

Aus der US 2007/0255383 A1 ist eine Elektrode bekannt, die durch den Rücken im Endabschnitt der Wirbelsäule implantierbar ist. In der Schrift ist allgemein beschrieben, dass der Abstand zwischen Elektroden variieren kann.

Die US 2010/0100152 A1 beschreibt eine Elektrode zur Implantation im Gehirn, wobei auch hier allgemein beschrieben ist, dass der Abstand zwischen Elektroden variieren kann.

Zum weiteren Stand der Technik wird zudem noch die WO 2009/097224 A1 genannt.

Aus der US 2009/0088827 A1 ist eine implantierbare drahtförmige Sammelelektrode zur Stimulation des Herzens bzw. zum Anschluss eines Herzschrittmachers bekannt. Der Abstand zwischen der dritten und vierten Mantelsegmentelektrode ist wesentlich größer als der Abstand zwischen mindestens zwei anderen Mantelsegmentelektroden.

Ausgehend von dem vorgenannten Stand der Technik liegt der Erfindung die Aufgabe zugrunde, eine verbesserte Sammelelektrode zur Nervenstimulation anzugeben, die dem Operateur mehr Positionierungsspielraum lässt und/oder mit der verbesserte Stimulationsergebnisse erzielbar sind. Insbesondere soll die Sammelelektrode derart ausgebildet sein, dass auch bei einer nicht optimalen (exakten) Positionierung eine gute Nervenstimulation ermöglicht wird. Besonders bevorzugt ist es, wenn mit der Sammelelektrode gleichzeitig zwei Nerven stimulierbar sind. Ferner besteht die Aufgabe darin, ein System, umfassend einen Schrittmacher und eine wie zuvor beschrieben verbesserte Sammelelektrode anzugeben.

Hinsichtlich der Sammelelektrode wird die Aufgabe mit den Merkmalen des Anspruchs 1 gelöst.

Hinsichtlich des Systems wird die Aufgabe durch die Kombination eines Schrittmachers mit einer nach dem Konzept der Erfindung ausgebildeten Sammelelektrode gelöst.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Der Erfindung liegt der Gedanke zugrunde, dass der von einem der isolierenden Abschnitte bestimmte Axialabstand zwischen zwei der Mantelsegmentelektroden größer ist als ein (anderer) Axialabstand zwischen zwei Mantelsegmentelektroden. Anders ausgedrückt wird erfindungsgemäß von der im Stand der Technik vorgesehenen Gleichbeabstandung sämtlicher Mantelsegmentelektroden abgewichen, derart, dass die Axialerstreckung mindestens eines isolierenden Abschnitts größer ist als die Axialerstreckung eines anderen, vorzugsweise benachbarten, isolierenden Abschnitts. Ganz besonders bevorzugt ist es dabei, wenn die Axialerstreckung von mindestens zwei der übrigen (axialkürzeren) isolierenden Abschnitte, zumindest näherungsweise, gleich ist. Überraschenderweise werden mit einem nach dem Konzept der Erfindung ausgebildeten Sammelelektrode verbesserte Stimulationsergebnisse erzielt, d.h. die Wahrscheinlichkeit einer Linderung von Patientenbeschwerden wird erhöht. Mit der nach dem Konzept der Erfindung ausgebildeten Sammelelektrode ist eine selektivere Stimulierung von Nerven möglich, als mit den aus dem Stand der Technik bekannten Sammelelektroden. Auch ist eine nach dem Konzept der Erfindung ausgebildete Elektrode überraschend unempfindlicher gegenüber einer (geringen) Fehlpositionierung, d.h. eine (geringe) Fehlpositionierung hat einen geringeren Einfluss auf das Stimulationsergebnis, als dies bei Sammelelektroden nach dem Stand der Technik der Fall ist, bei denen sämtliche Mantelsegmentelektroden gleich beabstandet sind. Ein weiterer wesentlicher Vorteil der erfindungsgemäßen Sammelelektrode ist, dass diese - bei geeigneter Positionierung - selektiv zwei benachbarte Nerven gleichzeitig stimulieren kann, insbesondere indem zwei über den axial längeren isolierenden Abschnitt voneinander getrennte Gruppen von Mantelsegmentelektroden auf die zwei Nerven verteilt angeordnet werden. So ist mit einer derartig ausgebildeten Sammelelektrode beispielsweise eine selektive Pudendus-Stimulation und eine selektive Ischias-Stimulation mit einer einzigen Sammelelektrode möglich.

Die nach dem Konzept der Erfindung ausgebildete Sammelelektrode dient, wie vorerwähnt, in erster Linie zur Neurostimulation, indem die Sammelelektrode bzw. die Mantelsegmentelektroden der Sammelelektrode mit einem bestimmten Stimulationsmuster mittels eines Schrittmachers beaufschlagt wird. Bevorzugt wird das Stimulationsmuster in Abhängigkeit der zu behandelnden Indikation gewählt. Die nach dem Konzept der Erfindung ausgebildete Sammelelektrode kann alternativ auch als Sensor zum Abgreifen von Nervenimpulsen eingesetzt werden. Die nach dem Konzept der Erfindung ausgebildete Sammelelektrode eignet sich bevorzugt zum Einsatz bei folgenden Indikationen:
- Neurogene oder nicht neurogene Harnblasenüberaktivität (Sakral- und Pudendal-Nervstimulation)
- Neurogene oder nicht neurogene, myogene Harnblasen-Hypo-/Atonie
- Pudendus Blockade (bei Querschnittslähmung)
- Blasen-/Darmentleerung bei Hyperaktivität Deblockade (bei Querschnittslähmung)
- Spastizität in den unteren Extremitäten, insbesondere bei Multipler Sklerose, Polyneuropatie, Tetra/Paraplegie, etc. (Ischiasnervstimulation)
- Errektions- und Sexualprobleme, Errektionsverlust, Ejakulationsunvermögen, vorzeitige Ejakulation (Ischiasnervenwurzelstimulation/Pudendalnervstimulation)
- Orgasmusunfähig bei Frauen
- Neurogene und nicht nicht neurogene Harnblasen/Rektum-Inkontinenz (Pudendalnervstimulation)
- Chronische Verstopfungen
- Verschiedene Pathologien und Beschwerden (gleichzeitige Stimulation verschiedener - mindestens zwei - Nerven)
- Pudendal Neuralgie => Neuralgie Ischias Nerv

Besonders bevorzugt ist es, wenn die Sammelelektrode drahtförmig ausgebildet ist. Unter drahtförmig ist dabei eine langgestreckte, beispielsweise stabförmige, starre oder alternativ, gegebenenfalls verformbare Ausformung zu verstehen. Bevorzugt ist die Axialerstreckung der, insbesondere drahtförmigen, Sammelelektrode aus einem Wertebereich zwischen etwa 45mm und etwa 65mm gewählt. Besonders bevorzugt beträgt die Axialerstreckung etwa 57mm. Dabei bezieht sich das Längenmaß auf den Abstand der voneinander abgewandten axialen Enden der am weitesten voneinander entfernten Mantelsegmentelektroden. Besonders zweckmäßig ist es, wenn der Durchmesser der, bevorzugt zumindest näherungsweise zylindrisch, insbesondere kreiszylindrisch, konturierten Sammelelektrode aus einem Wertebereich zwischen 0,5mm und 2mm, ganz besonders bevorzugt aus einem Wertebereich zwischen 0,8mm und 1,2mm gewählt ist. Noch weiter bevorzugt beträgt der Durchmesser etwa 1 mm.

Ganz besonders bevorzugt ist es, wenn nicht nur die Axialerstreckung eines einzigen zwischen zwei der Mantelsegmentelektroden angeordneten isolierenden Abschnittes größer ist als die Axialerstreckung mindestens eines anderen zwischen zwei der Mantelsegmentelektroden angeordneten isolierenden Abschnittes, sondern wenn die Axialerstreckung von mindestens zwei, oder von ausschließlich zwei, isolierenden Abschnitten, oder von mindestens drei, oder von ausschließlich drei, isolierenden Abschnitten, oder von mindestens vier, oder von ausschließlich vier, isolierenden Abschnitten, die jeweils zwischen zwei Mantelsegmentelektroden angeordnet sind, größer ist als die Axialerstreckung mindestens eines anderen isolierenden, zwischen zwei Mantelsegmentelektroden angeordneten isolierenden Abschnittes. Ganz besonders bevorzugt ist es, wenn die Axialerstreckung der isolierenden Abschnitte, die eine größere Axialerstreckung aufweisen als mindestens ein anderer isolierender Abschnitt, zumindest näherungsweise, gleich groß ist. Bevorzugt ist die Axialerstreckung mindestens eines der isolierenden Abschnitte mit einer größeren Axialerstreckung, vorzugsweise sämtlicher isolierender Abschnitte mit einer größeren Axialerstreckung, aus einem Wertebereich zwischen etwa 4mm und etwa 8mm, ganz besonders bevorzugt zwischen etwa 5mm und etwa 7mm gewählt. Ganz besonders bevorzugt beträgt die Axialerstreckung etwa 6mm.

Besonders zweckmäßig ist es, wenn die Axialerstreckung mindestens eines isolierenden Abschnittes mit einer geringeren Axialerstreckung aus einem Wertebereich zwischen etwa 2mm und etwa 4mm gewählt ist. Ganz besonders bevorzugt beträgt die Axialerstreckung etwa 3mm. Besonders zweckmäßig ist es, wenn mindestens zwei isolierende Abschnitte mit einer geringeren Axialerstreckung die gleiche Axialerstreckung, vorzugsweise aus dem vorgenannten Wertebereich, aufweisen. Bevorzugt sind insgesamt drei isolierende Abschnitte mit einer geringen Axialerstreckung, insbesondere mit der identischen Axialerstreckung, vorzugsweise aus dem vorgenannten Wertebereich, vorgesehen.

In Weiterbildung der Erfindung ist mit Vorteil vorgesehen, dass die Axialerstreckung der isolierenden Abschnitte mit einer größeren Axialerstreckung, zumindest näherungsweise, gleich groß ist und/oder die Axialerstreckung der isolierenden Abschnitte mit einer geringeren Axialerstreckung, zumindest näherungsweise, gleich groß ist.

In Weiterbildung der Erfindung ist mit Vorteil vorgesehen, dass die Sammelelektrode mindestens fünf axial über jeweils einen isolierenden Abschnitt voneinander beabstandete Mantelsegmentelektroden aufweist und dass, von einem ersten (insbesondere freien) Ende der Sammelelektrode her gezählt, die Axialerstreckung des die zweite und die dritte Mantelsegmentelektrode voneinander isolierenden Abschnitts oder die Axialerstreckung des isolierenden Abschnitts zwischen der dritten und der vierten Mantelsegmentelektrode oder die Axialerstreckung zwischen der vierten und der fünften Mantelsegmentelektrode größer ist als die Axialerstreckung mindestens eines anderen isolierenden Abschnitts (zwischen zwei Mantelsegmentelektroden). Gemäß einer realisierbaren Ausführungsvariante handelt es sich bei dem isolierenden Abschnitt mit der größeren Axialerstreckung um den isolierenden Abschnitt zwischen zwei Mantelsegmentelektroden mit der größten Axialerstreckung, d.h. es existiert nicht ein isolierender Abschnitt zwischen zwei Mantelsegmentelektroden, der eine größere und/oder gleich große Axialerstreckung aufweist. Wie jedoch bereits erläutert ist es möglich und bevorzugt mindestens zwei gleich lange isolierende Abschnitte vorzusehen, die eine größere Axialerstreckung aufweisen als mindestens ein isolierender, zwischen zwei Mantelsegmentelektroden angeordneter Abschnitt. Besonders zweckmäßig ist es, den Abstand zwischen der zweiten und der dritten Mantelsegmentelektrode oder zwischen der dritten oder vierten Mantelsegmentelektrode oder zwischen der vierten und der fünften Mantelsegmentelektrode größer zu wählen als den Axialabstand zwischen zwei geringer beabstandeten Mantelsegmentelektroden. Besonders gute Ergebnisse wurden mit einer Sammelelektrode erzielt, die insgesamt acht Mantelsegmentelektroden aufweist und bei der der Abstand zwischen der dritten und der vierten Mantelsegmentelektrode größer ist als der Abstand zwischen mindestens zwei anderen Mantelsegmentelektroden.

Besonders bevorzugt ist eine Ausführungsvariante der Sammelelektrode, bei der diese mindestens acht, vorzugsweise ausschließlich acht kontaktierte, Mantelsegmentelektroden aufweist, wobei jeweils zwei benachbarte Mantelsegmentelektroden über einen isolierenden Abschnitt voneinander getrennt sind. Dabei ist es besonders bevorzugt, wenn, gezählt ausgehend von einem ersten Ende der Mantelsegmentelektroden, der isolierende Abschnitt zwischen der dritten und der vierten Mantelsegmentelektrode und/oder die Axialerstreckung des isolierenden Abschnittes zwischen der fünften und der sechsten Mantelsegmentelektrode und/oder die Axialerstreckung des isolierenden Abschnittes zwischen der sechsten und der siebten Mantelsegmentelektrode und/oder die Axialerstreckung des isolierenden Abschnittes zwischen der siebten und der achten Mantelsegmentelektrode größer ist als die Axialerstreckung mindestens eines der isolierenden Abschnitte der Sammelelektrode. Ganz besonders bevorzugt ist die Axialerstreckung mindestens eines der vorgenannten isolierenden Abschnitte mit einer größeren Axialerstreckung, ganz besonders bevorzugt sämtlicher vorgenannter isolierender Abschnitte mit einer größeren Axialerstreckung größer als die Axialerstreckung des isolierenden Abschnittes zwischen der ersten und der zweiten Mantelsegmentelektrode und/oder größer als die Axialerstreckung des isolierenden Abschnittes zwischen der zweiten und der dritten Mantelsegmentelektrode und/oder als die Axialerstreckung des isolierenden Abschnittes zwischen der vierten und der fünften Mantelsegmentelektrode. Ganz besonders bevorzugt ist die Axialerstreckung der vorgenannten isolierenden Abschnitte mit einer geringeren Axialerstreckung gleich groß. Ganz besonders bevorzugt beträgt die Axialerstreckung der isolierenden Abschnitte mit der geringeren Axialerstreckung zumindest näherungsweise der Axialerstreckung mindestens einer Mantelsegmentelektrode, ganz besonders bevorzugt sämtlicher Mantelsegmentelektroden.

Ganz besonders bevorzugt ist es, wenn die Axialerstreckung mindestens eines eine größere Axialerstreckung aufweisenden isolierenden Abschnittes, vorzugsweise sämtlicher Axialabschnitte mit einer größeren Axialerstreckung, um einen Faktor aus einem Wertebereich zwischen etwa 1,5 und etwa 3, vorzugsweise von etwa 2, mal größer ist als die Axialerstreckung mindestens eines anderen isolierenden Abschnittes, vorzugsweise als die Axialerstreckung sämtlicher, insbesondere gleich langer anderer isolierender Abschnitte mit einer geringeren Axialerstreckung.

Bevorzugt ist die Axialerstreckung der Mantelsegmentelektroden aus einem Wertebereich zwischen etwa 2mm und etwa 4mm gewählt. Mit Vorteil beträgt sie etwa 3mm. Bevorzugt ist die Axialerstreckung des mindestens einen axial längeren isolierenden Abschnitts aus einem Wertebereich zwischen 5mm und 7mm gewählt, bevorzugt beträgt sie etwa 6mm. Mit Vorteil ist die Axialerstreckung der anderen (kürzeren) isolierenden Abschnitte aus einem Wertebereich zwischen etwa 2mm und etwa 4mm gewählt, bevorzugt beträgt sie etwa 3mm.

Wie bereits erwähnt, ist eine Ausführungsform mit insgesamt acht Mantelsegmentelektroden besonders bevorzugt. Es sind auch Ausführungsvarianten mit 6, 7, 9, 10 oder gegebenenfalls sogar mehr als 10 Sammelelektroden realisierbar.

Wie eingangs erwähnt, ist es besonders bevorzugt, wenn jede Mantelsegmentelektrode einzeln und/oder in Gruppen elektrisch kontaktierbar/kontaktiert, insbesondere ansteuerbar, ist. Um dies zu ermöglichen, ist in Weiterbildung der Erfindung mit Vorteil vorgesehen, dass zu jeder Mantelsegmentelektrode oder zu jeder Gruppe von Mantelsegmentelektroden, vorzugsweise im Innern der Sammelelektrode, eine die jeweilige Mantelsegmentelektrode kontaktierende elektrische Leitung geführt ist, wobei die die Mantelsegmentelektroden kontaktierenden elektrischen Leitungen voneinander elektrisch isoliert sind, um einen Kurzschluss zu vermeiden. Bevorzugt sind die Leitungen in einem gemeinsamen Anschlusskabel zusammengefasst, welches die Sammelelektrode mit einem Schrittmacher verbindet. Bevorzugt ist der Durchmesser des Anschlusskabels aus einem Wertebereich zwischen etwa 0,5mm und 2mm, vorzugsweise zwischen 0,8mm und 1,2mm, gewählt. Bevorzugt beträgt der Durchmesser etwa 1 mm. Bevorzugt entspricht der Durchmesser des Anschlusskabels, zumindest näherungsweise, dem Durchmesser der Sammelelektrode.

Bevorzugt ist das Anschlusskabel weiter entfernt von dem weiter zuvor diskutierten ersten, insbesondere distalen, Ende der Sammelelektrode als zu einem von dem ersten axialen Ende abgewandten zweiten axialen Ende. Anders ausgedrückt mündet das Anschlusskabel in die Sammelelektrode an einer Stelle, die weiter von dem ersten als von dem zweiten Ende beabstandet ist; mit Vorteil befindet sich diese Mündungsstelle am zweiten Ende. Beispielweise tritt das Anschlusskabel axial in die Sammelelektrode ein.

Ganz besonders bevorzugt ist Ausführungsvariante, bei der die Sammelelektrode endseitig an dem Anschlusskabel angeordnet ist. Dies bedeutet, dass die eigentliche Sammelelektrode, d.h. die Anordnung der Mantelsegmentelektroden das Anschlusskabel axial endseitig abschließt bzw. einen Endabschnitt des Anschlusskabels bildet. Somit befindet sich die Sammelelektrode nicht an einer beliebigen Axialposition am Anschlusskabel, sondern ausdrücklich axial endseitig, um so die Sammelelektrode optimal, insbesondere durch Angreifen am Anschlusskabel, positionieren zu können. Die Sammelelektrode bildet also die endseitige Fortsetzung des Anschlusskabels bzw. des Anschlusskabelendes, woraus eine drahtförmige Ausbildung der Sammelelektroden-Anschlusskabelanordnung resultiert. Bevorzugt entspricht der Durchmesser des Anschlusskabels, zumindest näherungsweise (± 20%, vorzugsweise ± 10%), dem Durchmesser der, vorzugsweise von dem Endabschnitt des Anschlusskabels gebildeten, Sammelelektrode.

Im Hinblick auf die metrische Ausgestaltung der isolierenden Abschnitte und/oder der Mantelsegmentelektroden gibt es unterschiedliche Möglichkeiten. Diese können sich beispielsweise nur über Umfangsabschnitte erstrecken. Besonders bevorzugt ist es jedoch, wenn es sich bei den isolierenden Abschnitten und/oder den Mantelsegmentelektroden um umfangsgeschlossene Ringsegmente handelt.

Die Erfindung führt auch auf ein System, umfassend eine nach dem Konzept der Erfindung ausgebildete Sammelelektrode, die elektrisch leitend über eine Anschlusskabel, vorzugsweise ebenfalls implantierbaren, Schrittmacher verbunden ist, der bevorzugt derart ausgebildet ist, dass die Sammelelektrode bzw. die einzelnen Mantelsegmentelektroden mit einem Stimulationsmuster beaufschlagbar sind. Bevorzugt kann aus vorgegebenen Stimulationsmustern, insbesondere durch den Operateur oder einem Medizintechniker ausgewählt werden. Der Schrittmacher umfasst bevorzugt eine Energiequelle und eine Steuereinheit, mit der Mantelsegmentelektroden auf ein unterschiedliches elektrisches Potential legbar sind.

Besonders bevorzugt handelt es sich um einen acht-kanaligen Schrittmacher.

Als besonders zweckmäßig hat es sich herausgestellt, wenn die distalen drei Mantelsegmentelektroden, also die drei ersten Mantelsegmentelektroden gezählt vom ersten Ende der Sammelelektrode mit Masse, Pluspotential bzw. ansteuerbar sind, insbesondere zu dem Zweck den Nervus pudendus tripolar stimulieren zu können und somit die Selektionsmöglichkeit dieser Simulation im Vergleich zum Stand der Technik noch zu erweitern. Eine derartige Ausführungsform würde zudem die Stimulation des gesamten sakralen Plexus, vom Truncus lumbosacralis bis S5 mit einer einzigen Elektrode ermöglichen.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1:: ein System, umfassend einen über ein Anschlusskabel mit einer Sammelelektrode verbundenen Schrittmacher,
- Fig. 2:: eine detaillierte Darstellung der Ausführungsvariante der Sammelelektrode gemäß Fig. 1,
- Fig. 3:: eine Darstellung einer implantierten bei gleichzeitig auf zwei Nerven angeordneten Sammelelektrode, und
- Fig. 4: eine bevorzugte Ausführungsvariante einer Sammelelektrode mit acht Mantelsegmentelektroden.

In den Figuren sind gleiche Elemente und Elemente mit der gleichen Funktion mit den gleichen Bezugszeichen gekennzeichnet.

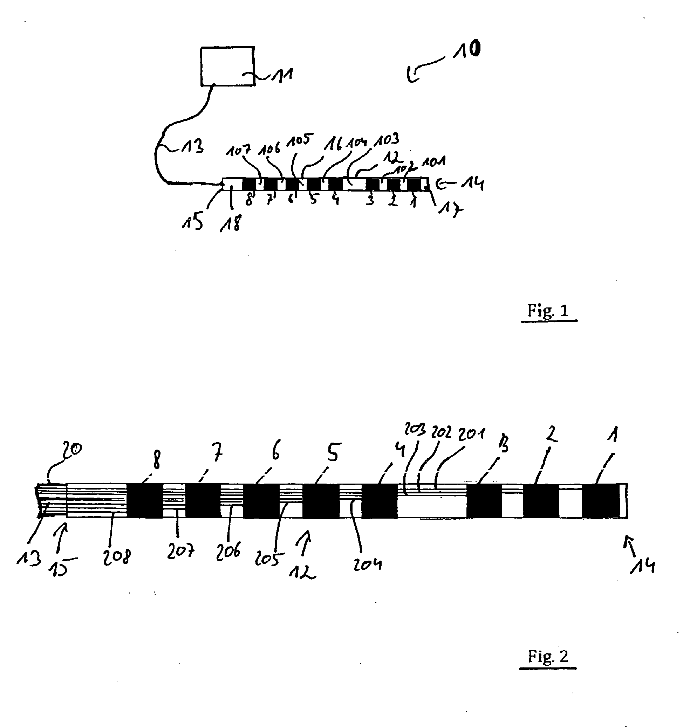

In Fig. 1 ist ein System 10 zur Neurostimulation von Nerven in einer stark schematisierten Ansicht. Das System 10 umfasst einen implantierbaren, hier acht-kanaligen, Schrittmacher 11, der zeichnerisch im Vergleich zu einer ebenfalls im System 10 umfassten Sammelelektrode 12 wesentlich zu klein dargestellt ist. Der Schrittmacher 11 ist über ein Anschlusskabel 13, welches, wie später noch erläutert werden wird, mehrere elektrisch voneinander isolierte Leitungen umfasst, mit der Sammelelektrode 12 verbunden. Das Anschlusskabel 13 mündet in ein von einem ersten, distalen Ende 14 abgewandtes zweites axiales Ende 15 der drahtförmigen, im Wesentlichen als langgestreckter Zylinderkörper konturierten Sammelelektrode 12.

Die im Wesentlichen zylindrische Mantelfläche 16 der Sammelelektrode 12 umfasst in dem gezeigten, bevorzugten Ausführungsbeispiel insgesamt acht Mantelsegmentelektroden 1 bis 8 aufsteigend gezählt ausgehend von dem ersten Ende 14. Die Mantelsegmentelektroden 1 bis 8 sind einzeln mittels des Schrittmachers 11 ansteuerbar und sind elektrisch voneinander isoliert. Hierzu befindet sich zwischen jeweils zwei der axial hintereinander angeordneten Mantelsegmentelektroden 1, 2; 2, 3; 3, 4; 4, 5; 5, 6; 6, 7; 7, 8 jeweils ein isolierender Abschnitt 101 bis 107, ebenfalls aufsteigend gezählt, ausgehend vom ersten Ende 14 der Sammelelektrode 12. Aus Fig. 1 ist zu erkennen, dass die Axialerstreckung des, gezählt ausgehend vom ersten Ende 14 dritten isolierenden Abschnitts 103 größer ist, in dem gezeigten Ausführungsbeispiel etwa doppelt so groß ist, wie die Axialerstreckungen sämtlicher anderer isolierender Abschnitte 101, 102 und 104 bis 107. Diese übrigen isolierenden Abschnitte 101, 102 und 104 bis 107 weisen die gleiche Axialerstreckung auf, von in diesem Ausführungsbeispiel etwa 3mm. Aus Fig. 1 ist weiter zu erkennen, dass die umfangsgeschlossenen, ringsegmentförmigen Mantelsegmentelektroden 1 bis 8 alle gleich groß dimensioniert sind und alle die gleiche Axialerstreckung von in dem gezeigten Ausführungsbeispiel 3mm aufweisen. Die Axialersteckung der gesamten Sammelelektrode 12 beträgt in dem gezeigten Ausführungsbeispiel 57mm. Der Durchmesser beträgt 1 mm.

Aus Fig. 1 ergibt sich weiterhin, dass in der Zeichnungsebene rechts von der ersten Mantelelektrode ein erster isolierender Endabschnitt 17 vorgesehen ist, der von einem das zweite Ende 15 bildenden zweiten Endabschnitt 18 beabstandet und abgewandt angeordnet ist.

Durch den größeren Abstand zwischen der dritten und vierten Mantelsegmentelektrode 16 werden die erfindungsgemäßen Vorteile erreicht.

Fig. 2 zeigt in einer im Vergleich zu Fig. 1 vergrößerten Darstellung einer Sammelelektrode 12. Diese umfasst auch acht Mantelsegmentelektroden 1 bis 8 und ist auch ansonsten ausgebildet, wie die in Fig. 1 dargestellte Sammelelektrode 12.

Aus der schematischen Darstellung gemäß Fig. 2 ergibt sich, dass jede Mantelsegmentelektrode 1 bis 8 einzeln kontaktiert ist mit einer isolierten elektrischen (Ansteuer-) Leitung 201 bis 208, wobei alle Leitungen 201 bis 208 aus der Sammelelektrode 12 herausgeführt sind, und zwar in dem gezeigten Ausführungsbeispiel am zweiten, proximalen Ende 15. Bis dorthin sind sie im Inneren der Sammelelektrode mit Radialabstand zur Umfangswand (Mantelfläche) geführt. Die Leitungen 201 bis 208 vereinigen sich zu einem einzigen mit einem Mantel 20 versehenen Anschlusskabel 13 zur Kontaktierung des in Fig. 2 nicht dargestellten Schrittmachers.

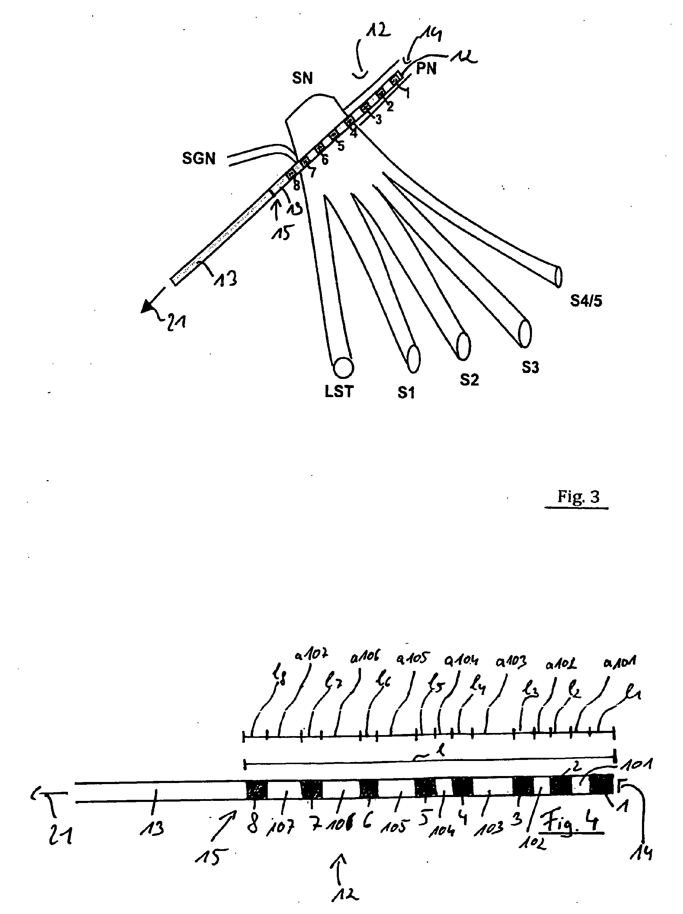

Fig. 3 zeigt eine bevorzugte Anordnung einer nach dem Konzept der Erfindung ausgebildeten Sammelelektrode 12 im menschlichen Körper. Diese Sammelelektrode 12 befindet sich mit den ersten drei Mantelsegmentelektroden 1 bis 3 (erste Gruppe) auf dem Nervus pudendus (PN), wohingegen sich die weiteren Mantelsegmentelektroden 4 bis 8 (zweite Gruppe) quer über den gesamten sakralen Plexus - vom Truncus lumbosacralis bis zum S5 erstrecken.

Das am zweiten Ende 15 axial herausgeführte Anschlusskabel 13 führt zu dem Schrittmacher (Generator) mit dem die Gesamtheit der Mantelsegmentelektroden 1 bis 8 mit einem geeigneten Stimulationsmuster beaufschlagbar ist. Der Pfeil 21 deutet die Lage des nicht gezeigten Schrittmachers an.

Die in Fig. 3 dargestellten Abkürzungen stehen im Einzelnen für:
- SN:: Ischiasnerv
- PN:: Nervus Pudendus
- SGN:: Superior Gluteal Nerv
- LST:: Lumbo Sakral Truncus
- S1:: Sakralnervenwurzel 1
- S2:: Sakralnervenwurzel 2
- S3:: Sakralnervenwurzel 3
- S4/5:: Sakralnervenwurzel 4/5

In Fig. 4 ist die am meisten bevorzugte Ausführungsvariante einer Sammelelektrode 12 gezeigt, die über ein Anschlusskabel 13, an dessen axialen Ende die Sammelelektrode 12 ausgebildet ist, an einen, vorzugsweise achtkanaligen Schrittmacher, wie dieser beispielhaft in Fig. 1 dargestellt ist, anschließbar ist. Auf die Darstellung der in Fig. 2 gezeigten elektrischen Anschlussleitungen innerhalb des Anschlusskabels 13 bzw. innerhalb der Sammelelektrode 12 zur Kontaktierung der Mantelsegmentelektroden 1 bis 8 wurde aus Übersichtlichkeitsgründen verzichtet. Grundsätzlich können die Mantelsegmentelektroden 1 bis 8 bei dem Ausführungsbeispiel gemäß Fig. 4 analog zu Fig. 2 kontaktiert sein.

In dem gezeigten Ausführungsbeispiel beträgt die Länge, d.h. die Axialerstreckung I der Sammelelektrode 12, gemessen zwischen den voneinander abgewandten Enden der am weitesten voneinander entfernten Mantelsegmentelektroden 1 und 8, 57mm.

Die Sammelelektrode 12 umfasst, mittels eines Schrittmachers einzeln oder in Gruppen ansteuerbare Mantelsegmentelektroden 1 bis 8, wobei die Mantelsegmentelektroden 1 bis 8 alle gleich lang sind, d.h. die gleiche Axialerstreckung I1 bis 18, von in dem gezeigten Ausführungsbeispiel etwa 3mm aufweisen.

Aus Fig. 4 ist zu erkennen, dass zwischen jeweils zwei benachbarten Mantelsegmentelektroden 1, 2; 2, 3; 3, 4; 4, 5; 5, 6; 6, 7; 7, 8 ein isolierender Abschnitt 101 bis 107 angeordnet ist, der die jeweils nebeneinander angeordneten, d.h. unmittelbar benachbarten Mantelsegmentelektroden elektrisch voneinander isoliert bzw. einen Kurzschluss verhindert. Insgesamt sind zwei Arten derartiger isolierender Abschnitte vorgesehen, nämlich die isolierenden Abschnitte 103, 105, 106, 107 mit einer größeren Axialerstreckung und die isolierenden Abschnitte 101, 102 und 104 mit einer kleineren Axialerstreckung. In dem gezeigten Ausführungsbeispiel entspricht die Axialerstreckung der isolierenden Abschnitte 101, 102, 104 mit der kleineren Axialerstreckung a101, a102 und a104 der Axialerstreckung I1 bis I8 der Mantelsegmentelektroden 1 bis 8, also in dem gezeigten Ausführungsbeispiel 3mm. Die Axialerstreckung der isolierenden Abschnitte 101, 102, 104 mit der geringeren Axialerstreckung a101, a102 und a104 ist in dem gezeigten Ausführungsbeispiel gleich groß. Gezählt sind die Mantelsegmentelektroden 1 bis 8 sowie die isolierenden Abschnitte 101 bis 107 ausgehend von dem (freien) ersten Ende 14 der Sammelelektrode 12.

Die Axialerstreckung a103, a105, a106, a107 jedes der isolierenden Abschnitte 103, 105, 106 und 107 entspricht der doppelten Axialerstreckung a101, a102, a104 von einem der isolierenden Abschnitte 101, 102, 104.

Zwischen der ersten und der zweiten Mantelsegmentelektrode 1, 2 befindet sich also ein isolierender Abschnitt 101 mit einer geringen Axialerstreckung a101. Ebenso befindet sich ein solcher isolierender Abschnitt 102 mit einer geringen Axialerstreckung a102 zwischen der zweiten und der dritten Mantelsegmentelektrode 2, 3. Zwischen der dritten und der vierten Mantelsegmentelektrode 3, 4 befindet sich ein isolierender Abschnitt 103 mit einer großen Axialerstreckung a103, die der doppelten Axialerstreckung von einem der isolierenden Abschnitte 101, 102 entspricht.

Zwischen der vierten und der fünften Mantelsegmentelektrode befindet sich ein isolierender Abschnitt 104 mit einer geringen Axialerstreckung a104. Zwischen der fünften und der sechsten, zwischen der sechsten und der siebten sowie zwischen der siebten und der achten Mantelsegmentelektrode 5, 6; 6, 7; 7, 8 befindet sich jeweils ein isolierender Abschnitt 105, 106, 107 mit einer großen Axialerstreckung a105, a106 bzw. a107, wobei die Axialerstreckungen a103, a105, a106 und a107 in dem gezeigten Ausführungsbeispiel identisch sind und etwa der doppelten Axialerstreckung der isolierenden Abschnitte 101, 102, 104 mit einer geringen Axialerstreckung entsprechen.

Es auch eine (nicht gezeigte) Ausführungsvariante denkbar, bei der sich die Axialerstreckung von mindestens zwei isolierenden Abschnitten 103, 105, 106, 107 mit einer größeren Axialerstreckung unterscheidet. Ebenso ist es denkbar, dass sich die Axialerstreckung von mindestens zwei isolierenden Abschnitten 101, 102 und 104 mit einer geringeren Axialerstreckung unterscheidet.

Die in Fig. 4 gezeigte, besonders vorteilhafte Sammelelektrode kann gemäß einer ersten Alternative eingesetzt werden um die Sakralnervenwurzeln 1, 2, 3, 4/5 zu stimulieren oder um gleichzeitig den Nervus Pudendus sowie den Ischiasnerv, insbesondere zusammen mit dem Nervus Gluteus Iferio zu stimulieren.

### Bezugszeichenliste

- 1 - 8: Mantelsegmentelektroden
- 10: System
- 11: Schrittmacher
- 12: Sammelelektrode
- 13: Anschlusskabel
- 14: erstes Ende
- 15: zweites Ende
- 16: Mantelfläche
- 17: erster Endabschnitt
- 18: zweiter Endabschnitt
- 20: Mantel
- 21: Pfeil
- 101 - 107: Isolierende Abschnitte
- 201 - 208: elektrische Leitungen
- I: Axialerstreckung der Sammelelektrode
- a101 - a107: Axialerstreckungen von isolierenden Abschnitten
- I1 - I8: Axialerstreckungen von Mantelsegmentelektroden

## Patentansprüche

1. In den menschlichen Körper laparoskopisch implantierbare, drahtförmige, Sammelelektrode (12) zur Neurostimulation der Sakrainervenwurzein 1, 2, 3, 4/5 oder zur gleichzeit igen Stimulation des Pudendusnervs sowie des Ischiasnervs, umfassend mehrere einzeln und/oder in Gruppen kontaktierbare Mantelsegmentelektroden (1 - 8), die axial in Richtung der Längserstreckung der Sammelelektrode (12) hintereinander angeordnet sind, wobei axial zwischen jeweils zwei benachbarten Mantelsegmentelektroden (1 - 8) ein isolierender Abschnitt (101 - 107) zum elektrischen voneinander Isolieren der jeweils zwei benachbarten Mantelsegmentelektroden (1 - 8) angeordnet ist, wobei die Axialerstreckung von zwei der isolierenden Abschnitte (101 - 107) unterschiedlich ist, wobei mindestens fünf Mantelsegmentelektroden (1, 2, 3, 4, 5) vorgesehen sind, und wobei, von einem ersten Ende (14) der Sammelelektrode (12) her betrachtet, die Axialerstreckung des isolierenden Abschnitts (103) zwischen der dritten und der vierten Mantelsegmentelektrode (3, 4) größer ist als die Axialerstreckung mindestens eines anderen isolierenden Abschnitts (101, 102, 101 107), wobei die Axialerstreckung des einen isolierenden Abschnitts (103) zwischen der dritten und der vierten Mantelsegmentelektrode (3, 4) um einen Faktor aus einem Wertebereich zwischen 1,5 und 3 mal größer ist als die Axialerstreckung mindestens eines anderen isolierenden Abschnitts (101, 102, 104-107).

2. Sammelelektrode nach Anspruch 1,
**dadurch gekennzeichnet,**
**dass** mindestens oder ausschließlich zwei, oder mindestens oder ausschließlich drei, oder mindestens oder ausschließlich vier isolierende Abschnitte (103, 105, 106, 107) zwischen jeweils zwei Mantelsegmentelektroden (3, 4; 5, 6; 6, 7; 7, 8) vorgesehen sind, die eine größere Axialerstreckung aufweisen als mindestens ein anderer isolierender Abschnitt (101, 102, 104) zwischen zwei Mantelsegmentelektroden (1, 2; 2, 3; 4, 5), oder als mindestens oder ausschließlich zwei andere isolierende Abschnitte (101, 102, 104) zwischen jeweils zwei Mantelsegmentelektroden (1, 2; 2, 3; 4, 5), oder als mindestens oder ausschließlich drei isolierende Abschnitte (101, 102, 104) zwischen jeweils zwei Mantelsegmentelektroden (1, 2; 2, 3; 4, 5).

3. Sammelelektrode nach Anspruch 2,
**dadurch gekennzeichnet,**
**dass** mindestens zwei der isolierenden Abschnitte (103, 105, 106, 107) mit der größeren Axialerstreckung, vorzugsweise sämtliche isolierenden Abschnitte (103, 105, 106, 107) mit der größeren Axialerstreckung die gleiche Axialerstreckung aufweisen und/oder dass mindestens zwei der isolierenden Abschnitte (101, 102, 104) mit der geringeren Axialerstreckung, vorzugsweise sämtliche isolierenden Abschnitte (101, 102, 104) mit der geringeren Axialerstreckung die gleiche Axialerstreckung aufweisen.

4. Sammelelektrode nach einem der vorhergehenden Ansprüche,
**dadurch gekennzeichnet,**
**dass** mindestens oder ausschließlich acht Mantelsegmentelektroden (1-8) vorgesehen sind, und dass von dem ersten Ende (14) der Sammelelektrode (12) her betrachtet der isolierende Abschnitt (103) zwischen der dritten und der vierten Mantelsegmentelektrode (3, 4) und/oder der isolierende Abschnitt (105) zwischen der fünften und der sechsten Mantelsegmentelektrode (5, 6) und/oder der isolierende Abschnitt (106) zwischen der sechsten und der siebten Mantelsegmentelektrode (6, 7) und/oder der isolierende Abschnitt (107) zwischen der siebten und der achten Mantelsegmentelektrode (7, 8) eine größere Axialerstreckung aufweist/aufweisen als mindestens ein anderer zwischen zwei Mantelsegmentelektroden (1 - 8) angeordneter isolierender Abschnitt (101, 102, 104), vorzugsweise als der isolierende Abschnitt (101) zwischen der ersten und der zweiten Mantelsegmentelektrode (1, 2) und/oder als der isolierende Abschnitt (102) zwischen der zweiten und der dritten Mantelsegmentelektrode (2, 3) und/oder als der isolierende Abschnitt (104) zwischen der vierten und der fünften Mantelsegmentelektrode (4, 5).

5. Sammelelektrode nach einem der vorhergehenden Ansprüche,
**dadurch gekennzeichnet,**
**dass** die Axialerstreckung des mindestens einen isolierenden Abschnitts (101 - 107) mit der größeren Axialerstreckung um ein Maß aus einem Wertebereich zwischen 1,5 mm und 5 mm, vorzugsweise von 3 mm größer ist als die Axialerstreckung mindestens eines anderen isolierenden Abschnitts (101 - 107).

6. Sammelelektrode nach einem der vorhergehenden Ansprüche,
**dadurch gekennzeichnet,**
**dass** insgesamt sechs, sieben, neun, zehn oder bevorzugt acht Mantelsegmentelektroden (1 - 8) vorgesehen sind.

7. Sammelelektrode nach einem der vorhergehenden Ansprüche,
**dadurch gekennzeichnet,**
**dass** zu jeder Mantelsegmentelektrode (1 - 8) oder zu jeder, zwei oder mehr Mantelsegmentelektroden (1 - 8) umfassenden Gruppe von Mantelsegmentelektroden (1 - 8), vorzugsweise im Inneren der Sammelelektrode (12), eine die Mantelsegmentelektrode (1 - 8) bzw. die Gruppe kontaktierende elektrische Leitung (201 - 208) geführt ist, wobei die elektrischen Leitungen (201 - 208) voneinander elektrisch isoliert sind und bevorzugt gemeinsam in einem Anschlusskabel (13) zur Sammelelektrode (12) geführt sind.

8. Sammelelektrode nach Anspruch 7,
**dadurch gekennzeichnet,**
**dass** das Anschlusskabel (13) weniger weit von einem von dem ersten Ende (14) abgewandten zweiten Ende (15) als von dem ersten Ende (14) beabstandet ist, vorzugsweise aus dem zweiten Ende (15) herausgeführt ist.

9. Sammelelektrode nach einem der vorhergehenden Ansprüche 7 oder 8,
**dadurch gekennzeichnet,**
**dass** die Sammelelektrode (12) endseitig an dem Anschlusskabel (13) angeordnet ist.

10. Sammelelektrode nach einem der vorhergehenden Ansprüche,
**dadurch gekennzeichnet,**
**dass** die isolierenden Abschnitte (101 - 107) und/oder die Mantelsegmentelektroden (1 - 8) als, vorzugsweise umfangegeschlossene, insbesondere metallische, Ringsegmente ausgebildet sind.

11. System zur Neurostimulation von Nerven, umfassend eine Sammelelektrode (12) nach einem der vorhergehenden Ansprüche, deren Mantelsegmentelektroden (1 - 8) einzeln und/oder in Gruppen von einem Schrittmacher (11) elektrisch ansteuerbar sind.

12. System nach Anspruch 11,
**dadurch gekennzeichnet,**
**dass** der Schrittmacher (11), vorzugsweise gleichzeitig, die ausgehend vom ersten Ende (14) der Sammelelektrode (12) erste Mantelsegmentelektrode (1) mit einem Pluspotential, die zweite Mantelsegmentelektrode (2) mit Masse und die dritte Mantelsegmentelektrode (3) mit einem Pluspotential ansteuernd ausgebildet ist.

## Claims

1. Wire-shaped collector electrode (12) which can be implanted laparoscopically into the human body for the neurostimulation of the sacral nerve roots 1, 2, 3, 4/5 or for the simultaneous stimulation of the pudendal nerve and the sciatic nerve, comprising a plurality of sheathed electrode segments (1-8) which can be contacted individually and/or in groups and are arranged axially in succession in the direction of the longitudinal extension of the collector electrode (12), wherein an insulating portion (101-107) is arranged axially between each two adjacent sheathed electrode segments (1-8) to electrically insulate each of the two adjacent sheathed electrode segments (1-8) from each other, wherein the axial extension of two of the insulating portions (101-107) is different, wherein at least five sheathed electrode segments (1, 2, 3, 4, 5) are provided, and wherein, when viewed from a first end (14) of the collector electrode (12), the axial extension of the insulating portion (103) between the third and the fourth sheathed electrode segment (3, 4) is greater than the axial extension of at least one other insulating portion (101, 102, 104, 107), wherein the axial extension of the one insulating portion (103) between the third and the fourth sheathed electrode segment (3, 4) is greater than the axial extension of at least one other insulating portion (101, 102, 104-107) by a factor in a range between 1.5 and 3 times.

2. Collector electrode according to claim 1, **characterised in that** at least or exclusively two, or at least or exclusively three, or at least or exclusively four insulating portions (103, 105, 106, 107) are provided between each two sheathed electrode segments (3, 4; 5, 6; 6, 7; 7, 8), which insulating portions have a greater axial extension than at least one other insulating portion (101, 102, 104) between two sheathed electrode segments (1, 2; 2, 3; 4, 5), or than at least or exclusively two other insulating portions (101, 102, 104) between each two sheathed electrode segments (1, 2; 2, 3; 4, 5), or than at least or exclusively three insulating portions (101, 102, 104) between each two sheathed electrode segments (1, 2; 2, 3; 4, 5).

3. Collector electrode according to claim 2, **characterised in that** at least two of the insulating portions (103, 105, 106, 107) having the greater axial extension, preferably all insulating portions (103, 105, 106, 107) having the greater axial extension, have the same axial extension, and/or **in that** at least two of the insulating portions (101, 102, 104) having the smaller axial extension, preferably all insulating portions (101, 102, 104) having the smaller axial extension, have the same axial extension.

4. Collector electrode according to any of the preceding claims, **characterised in that** at least or exclusively eight sheathed electrode segments (1-8) are provided, and **in that**, when viewed from the first end (14) of the collector electrode (12), the insulating portion (103) between the third and the fourth sheathed electrode segment (3, 4) and/or the insulating portion (105) between the fifth and the sixth sheathed electrode segment (5, 6) and/or the insulating portion (106) between the sixth and the seventh sheathed electrode segment (6, 7) and/or the insulating portion (107) between the seventh and the eighth sheathed electrode segment (7, 8) has/have a greater axial extension than at least one other insulating portion (101, 102, 104) arranged between two sheathed electrode segments (1-8), preferably than the insulating portion (101) between the first and the second sheathed electrode segment (1, 2) and/or than the insulating portion (102) between the second and the third sheathed electrode segment (2, 3) and/or than the insulating portion (104) between the fourth and the fifth sheathed electrode segment (4, 5).

5. Collector electrode according to any of the preceding claims, **characterised in that** the axial extension of the at least one insulating portion (101-107) having the greater axial extension is greater than the axial extension of at least one other insulating portion (101-107) by a measurement in a range between 1.5 mm and 5 mm, preferably by 3 mm.

6. Collector electrode according to any of the preceding claims, **characterised in that** a total of six, seven, nine, ten or preferably eight sheathed electrode segments (1-8) are provided.

7. Collector electrode according to any of the preceding claims, **characterised in that** an electric line (201-208), which contacts a sheathed electrode segment (1-8) or the group, is guided to each sheathed electrode segment (1-8) or to each group of sheathed electrode segments (1-8) comprising two or more sheathed electrode segments (1-8), preferably inside the collector electrode (12), the electric lines (201-208) being electrically insulated from each other and preferably being guided together in a connecting cable (13) to the collector electrode (12).

8. Collector electrode according to claim 7, **characterised in that** the connecting cable (13) is closer to a second end (15) remote from the first end (14), than to the first end (14), and is preferably guided out from the second end (15).

9. Collector electrode according to either preceding claim 7 or claim 8, **characterised in that** the collector electrode (12) is arranged at the end of the connecting cable (13).

10. Collector electrode according to any of the preceding claims, **characterised in that** the insulating portions (101-107) and/or the sheathed electrode segments (1-8) are formed as ring segments which are preferably closed around their circumference and are, in particular, metallic.

11. System for the neurostimulation of nerves, comprising a collector electrode (12) according to any of the preceding claims, the sheathed electrode segments (1-8) of which can be electrically actuated, individually and/or in groups, by a pacemaker (11).

12. System according to claim 11, **characterised in that**, starting from the first end (14) of the collector electrode (12), the pacemaker (11) is formed to actuate, preferably simultaneously, the first sheathed electrode segment (1) using a positive potential, the second sheathed electrode segment (2) using mass and the third sheathed electrode segment (3) using a positive potential.

## Revendications

1. Electrode collectrice (12) en forme de fil, apte à être implantée dans le corps humain par laparoscopie et servant à la neurostimulation des racines 1, 2, 3, 4/5 du nerf sacré ou à la stimulation simultanée du nerf pudendal et du nerf ischial, l'électrode comprenant plusieurs électrodes (1-8) à enveloppe segmentée aptes à être mises en contact séparément et/ou en groupes et disposées axialement les unes derrière les autres dans la direction de l'extension longitudinale de l'électrode de collecte (12), une partie isolante (101-107) étant disposée axialement entre deux électrodes (1-8) à enveloppe segmentées voisines pour isoler électriquement l'une de l'autre les deux électrodes (1-8) à enveloppe segmentée voisines, deux des parties isolantes (101-107) ayant des extensions axiales différentes, au moins cinq électrodes (1, 2, 3, 4, 5) à enveloppe segmentée étant prévues, l'extension axiale de la partie isolante (103) située entre la troisième et la quatrième électrode (3, 4) à enveloppe segmentée depuis une première extrémité (14) de l'électrode de collecte (12) étant supérieure à l'extension axiale d'au moins une autre partie isolante (101, 102, 101-107), l'extension axiale de l'une des parties isolantes (103) située entre la troisième et la quatrième électrode (3, 4) à enveloppe segmentée étant supérieure d'un facteur compris dans la plage de 1,5 à 3 à l'extension axiale d'au moins une autre partie isolante (101, 102, 104-107).

2. Electrode de collecte selon la revendication 1, **caractérisée en ce qu'**au moins ou exactement deux, au moins ou exactement trois ou au moins ou exactement quatre parties isolantes (103, 105, 106, 107) sont prévues entre deux électrodes (3, 4; 5, 6; 6, 7; 7, 8) à enveloppe segmentée qui présentent une extension axiale plus grande qu'au moins une autre partie isolante (101, 102, 104) située entre deux électrodes (1, 2; 2, 3; 4, 5) à enveloppe segmentée ou qu'au moins ou exactement deux autres parties isolantes (101, 102, 104) situées entre deux électrodes (1, 2; 2, 3; 4, 5) à enveloppe segmentée ou qu'au moins deux ou exactement trois parties isolantes (101, 102, 104) situées entre deux électrodes (1, 2; 2, 3; 4, 5) à enveloppe segmentée.

3. Electrode de collecte selon la revendication 2, **caractérisée en ce que** moins deux des parties isolantes (103, 105, 106, 107) qui présentent une plus grande extension axiale et de préférence toutes les parties isolantes (103, 105, 106, 107) qui présentent une plus grande extension axiale ont la même extension axiale et/ou **en ce qu'**au moins deux des parties isolantes (101, 102, 104) qui présentent une plus petite extension axiale et de préférence toutes les parties isolantes (101, 102, 104) qui présentent une plus petite extension axiale ont la même extension axiale.

4. Electrode de collecte selon l'une des revendications précédentes, **caractérisée en ce qu'**au moins ou exactement huit électrodes (1-8) à enveloppe segmentée sont prévues, **en ce que** depuis la première extrémité (14) de l'électrode de collecte (12), l'électrode isolante (103) située entre la troisième et la quatrième électrode (3, 4) à enveloppe segmentée et/ou la partie isolante (105) située entre la cinquième et la sixième électrode (5, 6) à enveloppe segmentée et/ou la partie isolante (106) située entre la sixième et la septième électrode (6, 7) à enveloppe segmentée et/ou la partie isolante (107) située entre la septième et la huitième électrode (7, 8) à enveloppe segmentée présentent une extension axiale plus grande qu'au moins une autre partie isolante (101, 102, 104) disposée entre deux électrodes (1-8) à enveloppe segmentée, de préférence que la partie isolante (101) située entre la première et la deuxième électrode (1, 2) à enveloppe segmentée et/ou que la partie isolante (102) située entre la deuxième et la troisième électrode (2, 3) à enveloppe segmentée et/ou que la partie isolante (104) située entre la quatrième et la cinquième électrode (4, 5) à enveloppe segmentée.

5. Electrode de collecte selon l'une des revendications précédentes, **caractérisée en ce que** l'extension axiale de la ou des parties isolantes (101-107) qui présentent la plus grande extension axiale est supérieure à l'extension axiale d'au moins une autre partie isolante (101, 107) d'une valeur située dans une plage comprise entre 1,5 mm et 5 mm et de préférence de 3 mm.

6. Electrode de collecte selon l'une des revendications précédentes, **caractérisée en ce qu'**en tout six, sept, neuf, dix ou de préférence huit électrodes (1-8) à enveloppe segmentée sont prévues.

7. Electrode de collecte selon l'une des revendications précédentes, **caractérisée en ce qu'**un conducteur électrique (201-208) est conduit jusqu'à chaque électrode à enveloppe segmentée (1-8) ou jusqu'à chaque groupe comprenant deux ou plus de deux électrodes à enveloppe segmentée (1-8), de préférence à l'intérieur de l'électrode de collecte (12), lequel conducteur électrique est en contact avec l'électrode à enveloppe segmentée (1-8) ou avec le groupe, les conducteurs électriques (201-208) étant isolés électriquement les uns des autres et étant conduits de préférence ensemble dans un câble (13) qui assure le raccordement de l'électrode de collecte (12).

8. Electrode de collecte selon la revendication 7, **caractérisé en ce que** le câble de raccordement (13) est situé de préférence moins loin d'une deuxième extrémité (15) non tournée vers la première extrémité (14) que de la première extrémité (14) et part de préférence de la deuxième extrémité (15).

9. Electrode de collecte selon l'une des revendications 7 ou 8 qui précèdent, **caractérisé en ce que** l'extrémité de l'électrode de collecte (12) est disposée sur le câble de raccordement (13).

10. Electrode de collecte selon l'une des revendications précédentes, **caractérisée en ce que** les parties isolantes (101-107) et/ou les électrodes (1-8) à enveloppe segmentée sont configurées comme segments annulaires, de préférence fermés à leur périphérie et en particulier métalliques.

11. Système de neurostimulation de nerfs comprenant une électrode de collecte (12) selon l'une des revendications précédentes dont les électrodes (1-8) à enveloppe segmentée peuvent être commandées électriquement séparément et/ou en groupes par un programmateur (11).

12. Système selon la revendication 11, **caractérisé en ce que** le programmateur (11) est configuré de manière à appliquer de préférence simultanément un potentiel positif sur la première électrode (1) à enveloppe segmentée qui part de la première extrémité (14) de l'électrode de collecte (12), à appliquer la masse sur la deuxième électrode (2) à enveloppe segmentée et à appliquer un potentiel positif sur la troisième électrode (3) à enveloppe segmentée.
